(19) Europäisches Patentamt
European Patent Office
Office européen des brevets

(11) Veröffentlichungsnummer: **0 547 461 A1**

(12) # EUROPÄISCHE PATENTANMELDUNG

(21) Anmeldenummer: **92120820.3**

(51) Int. Cl.5: **C07D 403/04**, A01N 43/64

(22) Anmeldetag: **07.12.92**

(30) Priorität: **18.12.91 DE 4141721**

(43) Veröffentlichungstag der Anmeldung:
**23.06.93 Patentblatt 93/25**

(84) Benannte Vertragsstaaten:
**BE CH DE ES FR GB IT LI NL**

(71) Anmelder: **BAYER AG**
**W-5090 Leverkusen 1 Bayerwerk(DE)**

(72) Erfinder: **Schallner, Otto, Dr.**
**Noldeweg 22**
**W-4019 Monheim(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Erdelen, Christoph, Dr.**
**Unterbüscherhof 22**
**W-5653 Leichlingen 3(DE)**

(54) **Substituierte Heterocyclyltriazindione als Herbizide und Insektizide.**

(57) Die Erfindung betrifft neue substituierte Heterocyclyltriazindione der allgemeinen Formel (I)

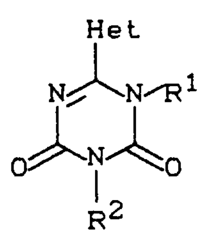

(I)

in welcher

$R^1$ für Wasserstoff oder für einen jeweils gegebenenfalls substituierten Rest der Reihe Alkyl, Alkenyl, Alkinyl oder Cycloalkyl steht,

$R^2$ für einen jeweils gegebenenfalls substituierten Rest der Reihe Alkyl, Alkenyl, Alkinyl oder Cycloalkyl steht und

Het für einen N-verknüpften, gegebenenfalls substituierten, ungesättigten, stickstoffhaltigen Heterocyclus steht, der gegebenenfalls weitere Heteroatome enthalten kann,

mehrere Verfahren zu ihrer Herstellung und ihre Verwendung als Herbizide und Insektizide.

EP 0 547 461 A1

Die Erfindung betrifft neue substituierte Heterocyclyltriazindione, mehrere Verfahren zu ihrer Herstellung und ihre Verwendung als Herbizide und Insektizide.

Es ist bekannt, daß bestimmte substituierte Triazindione wie beispielsweise die Verbindung 1-Isopropyl-4-[N-(2,2-dimethylpropyl)-acetamido]-1,3,5-(3H)-triazin-2,6-dion herbizide Eigenschaften besitzen (vergleiche z.B. DE 26 03 180).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue, substituierte Heterocyclyltriazindione der allgemeinen Formel (I),

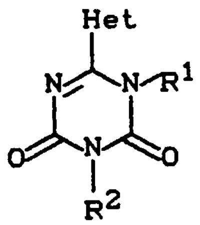

(I)

in welcher

R¹ für Wasserstoff oder für einen jeweils gegebenenfalls substituierten Rest der Reihe Alkyl, Alkenyl, Alkinyl oder Cycloalkyl steht,

$R^2$ für einen jeweils gegebenenfalls substituierten Rest der Reihe Alkyl, Alkenyl, Alkinyl oder Cycloalkyl steht und

Het für einen N-verknüpften, gegebenenfalls substituierten, ungesättigten, stickstoffhaltigen Heterocyclus steht, der gegebenenfalls weitere Heteroatome enthalten kann,

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Heterocyclyltriazindione der allgemeinen Formel (I)

erhält, wenn man

a) 4-Alkylthio-triazindione der Formel (II),

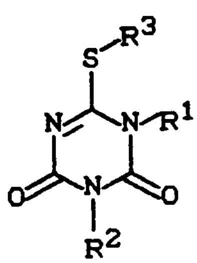

(II)

in welcher

$R^1$ und $R^2$ die oben angegebene Bedeutung haben und

$R^3$ für Alkyl steht,

mit Heterocyclen der Formel (III),

Het-H (III)

in welcher

Het die oben angegebene Bedeutung hat,

gegebenenfalls in Gegenwart eines Verdünnungsmittels umsetzt und gegebenenfalls anschließend

b) die so erhältlichen substituierten Heterocyclyltriazindione der Formel (I),

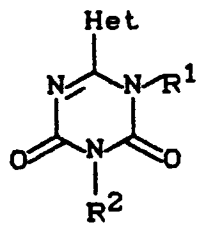

(I)

in welcher

$R^1$, $R^2$ und Het die oben angegebene Bedeutung haben,

mit elektrophilen Reagenzien im Heterocyclylteil des Substituenten Het gegebenenfalls in Gegenwart eines Verdünnungsmittels und gegebenenfalls in Gegenwart eines Reaktionshilfsmittels substituiert.

Schließlich wurde gefunden, daß die neuen substituierten Heterocyclyltriazindione der allgemeinen Formel (I) herbizide und insektizide Eigenschaften besitzen.

Überraschenderweise zeigen die erfindungsgemäßen substituierten Heterocyclyltriazindione der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten substituierten Triazindionen, wie beispielsweise die Verbindung 1-Isopropyl-4-[N-(2,2-dimethylpropyl)-acetamido]-1,3,5-(3H)-triazin-2,6-dion, welche chemisch und wirkungsmäßig naheliegende Verbindungen sind.

Die erfindungsgemäßen substituierten Heterocyclyltriazindione sind durch die Formel (I) allgemein definiert. Bevorzugt sind Verbindungen der Formel (I), bei welchen

$R^1$ für Wasserstoff, für einen jeweils geradkettigen oder verzweigten Rest der Reihe Alkyl mit 1 bis 12 Kohlenstoffatomen, Alkenyl mit 2 bis 6 Kohlenstoffatomen, Alkinyl mit 2 bis 6 Kohlenstoffatomen, Alkoxyalkyl oder Alkylthioalkyl mit jeweils 1 bis 6 Kohlenstoffatomen in den einzelnen Alkylteilen, Halogenalkyl mit 1 bis 6 Kohlenstoffatomen und 1 bis 13 gleichen oder verschiedenen Halogenatomen, Halogenalkenyl mit 2 bis 6 Kohlenstoffatomen und 1 bis 11 gleichen oder verschiedenen Halogenatomen, Halogenalkinyl mit 2 bis 6 Kohlenstoffatomen und 1 bis 9 gleichen oder verschiedenen Halogenatomen oder für gegebenenfalls einfach oder mehrfach, gleich oder verschieden substituiertes Cycloalkyl mit 3 bis 8 Kohlenstoffatomen steht, wobei als Cycloalkylsubstituenten infrage kommen: Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen,

$R^1$ außerdem für jeweils gegebenenfalls im Aryl- bzw. Heteroarylteil gegebenenfalls einfach oder mehrfach, gleich oder verschieden substituiertes Arylalkyl, Aryloxyalkyl, Arylthioalkyl oder Heteroarylalkyl mit jeweils 1 bis 6 Kohlenstoffatomen im geradkettigen oder verzweigten Alkylteil und jeweils 6 bis 10 Kohlenstoffatomen im Arylteil bzw. mit 2 bis 9 Kohlenstoffatomen und 1 bis 4 Heteroatomen - insbesondere Stickstoff, Sauerstoff und/oder Schwefel - im Heteroarylteil steht, wobei als Aryl bzw. Heteroarylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^2$ für einen jeweils geradkettigen oder verzweigten Rest der Reihe Alkyl, Alkoxy oder Alkylthio mit jeweils 1 bis 8 Kohlenstoffatomen, Alkenyl mit 2 bis 6 Kohlenstoffatomen, Alkinyl mit 2 bis 6 Kohlenstoffatomen, Halogenalkyl mit 1 bis 6 Kohlenstoffatomen und 1 bis 13 gleich oder verschiedenen Halogenatomen, Halogenalkenyl mit 2 bis 6 Kohlenstoffatomen und 1 bis 9 gleichen oder verschiedenen Halogenatomen, Halogenalkinyl mit 2 bis 6 Kohlenstoffatomen und 1 bis 9 gleichen oder verschiedenen Halogenatomen oder für gegebenenfalls einfach oder mehrfach, gleich oder verschieden substituiertes Cycloalkyl mit 3 bis 8 Kohlenstoffatomen steht, wobei als Cycloalkylsubstituenten infrage kommen: Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen,

$R^2$ außerdem für jeweils gegebenenfalls im Aryl- bzw. Heteroarylteil gegebenenfalls einfach oder

mehrfach, gleich oder verschieden substituiertes Arylalkyl, Aryloxyalkyl, Arylthioalkyl oder Heteroarylalkyl mit jeweils 1 bis 6 Kohlenstoffatomen im geradkettigen oder verzweigten Alkylteil und jeweils 6 bis 10 Kohlenstoffatomen im Arylteil bzw. mit 2 bis 9 Kohlenstoffatomen und 1 bis 4 Heteroatomen - insbesondere Stickstoff, Sauerstoff und/ oder Schwefel - im Heteroarylteil steht, wobei als Aryl- bzw. Heteroarylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl und

Het für einen N-verknüpften, gegebenenfalls einfach oder mehrfach, gleich oder verschieden substituierten, einfach oder mehrfach ungesättigten, stickstoffhaltigen Heterocyclus mit 2 bis 9 Kohlenstoffatomen und gegebenenfalls 1 bis 4 weiteren Heteroatomen - insbesondere Stickstoff, Sauerstoff und/oder Schwefel -steht, wobei als Heterocyclylsubstituenten die bei $R^2$ genannten infrage kommen.

Besonders bevorzugt sind Verbindungen der Formel (I), bei welchen

$R^1$ für Wasserstoff, für einen jeweils geradkettigen oder verzweigten Rest der Reihe Alkyl mit 1 bis 10 Kohlenstoffatomen, Alkenyl mit 2 bis 5 Kohlenstoffatomen, Alkinyl mit 2 bis 5 Kohlenstoffatomen, Alkoxyalkyl oder Alkylthioalkyl mit jeweils 1 bis 4 Kohlenstoffatomen in den einzelnen Alkylteilen, Halogenalkyl mit 1 bis 4 Kohlenstoffatomen und 1 bis 9 gleichen oder verschiedenen Halogenatomen, Halogenalkenyl mit 2 bis 5 Kohlenstoffatomen und 1 bis 9 gleichen oder verschiedenen Halogenatomen, Halogenalkinyl mit 2 bis 5 Kohlenstoffatomen und 1 bis 7 gleichen oder verschiedenen Halogenatomen oder für gegebenenfalls einfach bis fünffach, gleich oder verschieden substituiertes Cycloalkyl mit 3 bis 7 Kohlenstoffatomen steht, wobei als Cycloalkylsubstituenten infrage kommen:

Halogen, geradkettiges oder verzweigtes Alkyl mit 1 bis 3 Kohlenstoffatomen oder geradkettiges oder verzweigtes Halogenalkyl mit 1 bis 3 Kohlenstoffatomen und 1 bis 7 gleichen oder verschiedenen Halogenatomen,

$R^1$ außerdem für jeweils gegebenenfalls im Aryl- bzw. Heteroarylteil gegebenenfalls einfach bis fünffach, gleich oder verschieden substituiertes Arylalkyl, Aryloxyalkyl, Arylthioalkyl oder Heteroarylalkyl mit jeweils 1 bis 4 Kohlenstoffatomen im geradkettigen oder verzweigten Alkylteil und jeweils 6 oder 10 Kohlenstoffatomen im Arylteil bzw. mit 2 bis 9 Kohlenstoffatomen und 1 bis 3 Heteroatomen - insbesondere Stickstoff, Sauerstoff und/oder Schwefel - im Heteroarylteil steht, wobei als Aryl- bzw. Heteroarylsubstituenten jeweils infrage kommen:

Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 2 Kohlenstoffatomen und 1 bis 5 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis fünffach, gleich oder verschieden durch Halogen und/oder geradkettiges oder verzweigtes Alkyl mit 1 bis 3 Kohlenstoffatomen substituiertes Phenyl,

$R^2$ für einen jeweils geradkettigen oder verzweigten Rest der Reihe Alkyl, Alkoxy oder Alkylthio mit jeweils 1 bis 6 Kohlenstoffatomen, Alkenyl mit 2 bis 5 Kohlenstoffatomen, Alkinyl mit 2 bis 5 Kohlenstoffatomen, Halogenalkyl mit 1 bis 4 Kohlenstoffatomen und 1 bis 9 gleichen oder verschiedenen Halogenatomen, Halogenalkenyl mit 2 bis 5 Kohlenstoffatomen und 1 bis 9 gleichen oder verschiedenen Halogenatomen, Halogenalkinyl mit 2 bis 5 Kohlenstoffatomen und 1 bis 7 gleichen oder verschiedenen Halogenatomen oder für gegebenenfalls einfach bis fünffach, gleich oder verschieden substituiertes Cycloalkyl mit 3 bis 7 Kohlenstoffatomen steht, wobei als Cycloalkylsubstituenten infrage kommen:

Halogen, geradkettiges oder verzweigtes Alkyl mit 1 bis 3 Kohlenstoffatomen oder geradkettiges oder verzweigtes Halogenalkyl mit 1 bis 3 Kohlenstoffatomen und 1 bis 7 gleichen oder verschiedenen Halogenatomen,

$R^2$ außerdem für jeweils gegebenenfalls im Aryl- bzw. Heteroarylteil gegebenenfalls einfach bis fünffach, gleich oder verschieden substituiertes Arylalkyl, Aryloxyalkyl, Arylthioalkyl oder Heteroarylalkyl mit jeweils 1 bis 4 Kohlenstoffatomen im geradkettigen oder verzweigten Alkylteil und jeweils 6 oder 10 Kohlenstoffatomen im Arylteil bzw. mit 2 bis 9 Kohlenstoffatomen und 1 bis 3

Heteroatomen - insbesondere Stickstoff, Sauerstoff und/oder Schwefel - im Heteroarylteil steht, wobei als Aryl- bzw. Heteroarylsubstituenten jeweils infrage kommen:

Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 2 Kohlenstoffatomen und 1 bis 5 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis fünffach, gleich oder verschieden durch Halogen und/oder geradkettiges oder verzweigtes Alkyl mit 1 bis 3 Kohlenstoffatomen substituiertes Phenyl und

Het für einen N-verknüpften, gegebenenfalls einfach bis fünffach, gleich oder verschieden substituierten, einfach oder mehrfach ungesättigten, stickstoffhaltigen Heterocyclus mit 2 bis 9 Kohlenstoffatomen und gegebenenfalls 1 bis 3 weiteren Heteroatomen - insbesondere Stickstoff, Sauerstoff und/oder Schwefel - steht, wobei als Heterocyclylsubstituenten die bei $R^2$ genannten infrage kommen.

Ganz besonders bevorzugt sind Verbindungen der Formel (I), bei welchen

$R^1$ für Wasserstoff, für einen jeweils geradkettigen oder verzweigten Rest der Reihe Alkyl mit 1 bis 8 Kohlenstoffatomen, Alkenyl mit 2 bis 3 Kohlenstoffatomen, Alkinyl mit 2 bis 3 Kohlenstoffatomen, Alkoxyalkyl oder Alkylthioalkyl mit jeweils 1 bis 3 Kohlenstoffatomen in den einzelnen Alkylteilen, Halogenalkyl mit 1 oder 2 Kohlenstoffatomen und 1 bis 5 gleichen oder verschiedenen Halogenatomen, Halogenalkenyl mit 2 bis 3 Kohlenstoffatomen und 1 bis 5 gleichen oder verschiedenen Halogenatomen, Halogenalkinyl mit 2 bis 3 Kohlenstoffatomen und 1 bis 3 gleichen oder verschiedenen Halogenatomen oder für jeweils gegebenenfalls einfach bis dreifach, gleich oder verschieden substituiertes Cyclopropyl, Cyclopentyl oder Cyclohexyl steht, wobei als Substituenten jeweils infrage kommen:

Fluor, Chlor, Brom, Methyl, Ethyl oder Trifluormethyl,

außerdem für jeweils gegebenenfalls im Phenyl- bzw. Heteroarylteil gegebenenfalls einfach bis dreifach, gleich oder verschieden substituiertes Phenylalkyl, Phenyloxyalkyl, Phenylthioalkyl oder Heteroarylalkyl mit jeweils 1 bis 2 Kohlenstoffatomen im Alkylteil und gegebenenfalls 2 bis 9 Kohlenstoffatomen und 1 bis 3 Heteroatomen - insbesondere Stickstoff, Sauerstoff und/oder Schwefel - im Heteroarylteil steht, wobei als Phenyl- bzw. Heteroarylsubstituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n-oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl,

$R^2$ für einen jeweils geradkettigen oder verzweigten Rest der Reihe Alkyl, Alkoxy oder Alkylthio mit jeweils 1 bis 4 Kohlenstoffatomen, Alkenyl mit 2 bis 3 Kohlenstoffatomen, Alkinyl mit 2 bis 3 Kohlenstoffatomen, Halogenalkyl mit 1 bis 2 Kohlenstoffatomen und 1 bis 5 gleichen oder verschiedenen Halogenatomen, Halogenalkenyl mit 2 bis 3 Kohlenstoffatomen und 1 bis 5 gleichen oder verschieden Halogenatomen, Halogenalkinyl mit 2 bis 3 Kohlenstoffatomen und 1 bis 3 gleichen oder verschiedenen Halogenatomen oder für jeweils gegebenenfalls einfach bis dreifach, gleich oder verschieden substituiertes Cyclopropyl, Cyclopentyl oder Cyclohexyl steht, wobei als Substituenten jeweils infrage kommen:

Fluor, Chlor, Brom, Methyl, Ethyl oder Trifluormethyl,

$R^2$ außerdem für jeweils gegebenenfalls im Phenyl- bzw. Heteroarylteil gegebenenfalls einfach bis dreifach, gleich oder verschieden substituiertes Phenylalkyl, Phenyloxyalkyl, Phenylthioalkyl oder Heteroarylalkyl mit jeweils 1 bis 2 Kohlenstoffatomen im geradkettigen oder verzweigten Alkylteil und gegebenenfalls 2 bis 9 Kohlenstoffatomen und 1 bis 3 Heteroatomen - insbesondere Stickstoff, Sauerstoff und/oder Schwefel - im Heteroarylteil steht, wobei als Phenyl- bzw. Heteroarylsubstituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl und

Het für jeweils gegebenenfalls einfach bis dreifach, gleich oder verschieden substituiertes, jeweils N-verknüpftes Pyrazolyl, Pyrrolyl, Imidazolyl, Triazolyl, Tetrazolyl, Indolyl, Indazolyl oder Benzimidazolyl steht, wobei als Heterocyclylsubstituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

Verwendet man beispielsweise 1-Isopropyl-4-methylthio-1,3,5-(3H)-triazin-2,6-dion und Imidazol als Ausgangsstoffe, so läßt sich der Reaktionsablauf des erfindungsgemäßen Verfahrens (a) durch das folgende Formelschema darstellen:

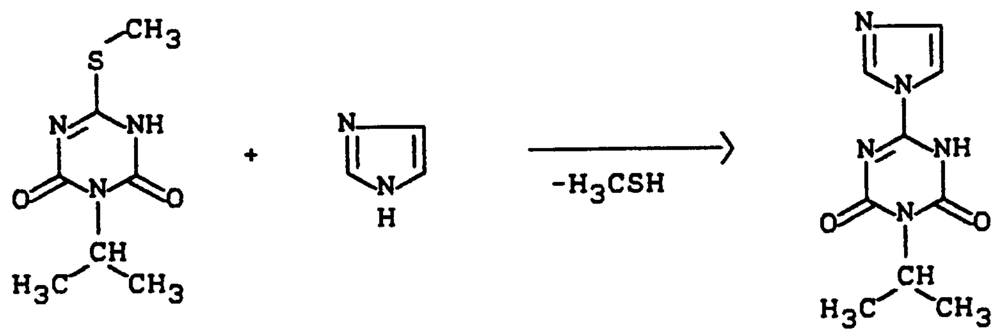


Verwendet man beispielsweise 1-Isopropyl-3-methyl-4-(1-pyrazolyl)-1,3,5-(3H)-triazin-2,6-dion und Salpetersäure als Ausgangsstoffe, so läßt sich der Reaktionsablauf des erfindungsgemäßen Verfahrens (b) durch das folgende Formelschema darstellen:

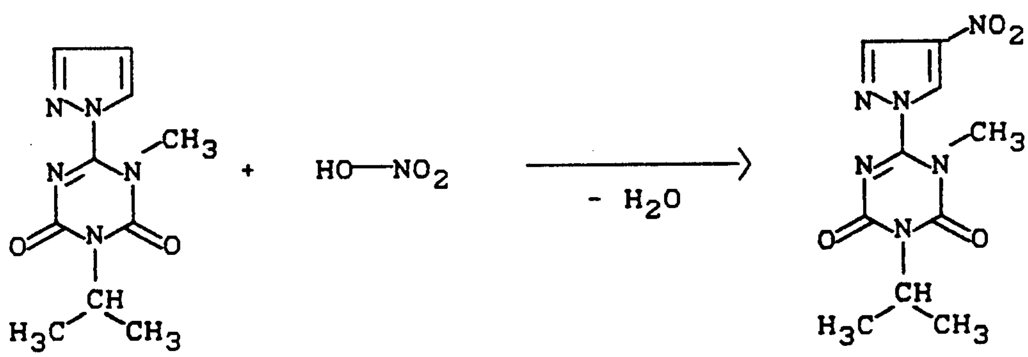


Die zur Durchführung des erfindungsgemäßen Verfahrens (a) als Ausgangsstoffe benötigten 4-Alkylthiotriazindione sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Verbindungen der Formel (I) als bevorzugt für diesen Substituenten genannt wurden.

$R^3$ steht vorzugsweise für geradkettiges oder verzweigtes Alkyl mit 1 bis 4 Kohlenstoffatomen, insbesondere für Methyl oder Ethyl.

Die 4-Alkylthio-triazindione der Formel (II) sind bekannt (vergleiche z. B. GB 14 35 585).

Die zur Durchführung des erfindungsgemäßen Verfahrens (a) weiterhin als Ausgangsprodukte erforderlichen Heterocyclen sind durch die Formel (III) allgemein definiert. In dieser Formel (III) steht Het vorzugsweise für diejenigen Reste, die bereits im Zusammenhang mit der Beschreibung der erfindungsgemäßen Stoffe der Formel (I) als bevorzugt für diesen Substituenten genannt wurden.

Die Heterocyclen der Formel (III) sind allgemein bekannte Verbindungen der organischen Chemie.

Die zur Durchführung des erfindungsgemäßen Verfahrens (b) weiterhin als Ausgangsstoffe benötigten elektrophilen Reagenzien sind allgemein bekannte Verbindungen der organischen Chemie. Geeignete elektrophile Reagenzien sind beispielsweise Halogenierungsmittel wie Sulfurylchlorid, Phosphoroxychlorid, Phosphoroxybromid oder Phosphortribromid oder Nitrierungsmittel wie Salpetersäure oder Nitriersäure.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische,

gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder organische Säuren, wie beispielsweise Eisessig.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 200 °C, vorzugsweise bei Temperaturen zwischen 50 °C und 150 °C.

Das erfindungsgemäße Verfahren (a) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (a) setzt man pro Mol 4-Alkylthio-triazindion der Formel (II) im allgemeinen 1,0 bis 50,0 Mol, vorzugsweise 1,0 bis 10,0 Mol Heterocyclus der Formel (III) ein. Die Reaktionsdurchführung, Aufarbeitung und Isolierung der Reaktionsprodukte erfolgt nach bekannten Verfahren (vergleiche hierzu auch die Herstellungsbeispiele).

Als Verdünnungsmittel zur Durchführung des erfindungsgemäßen Verfahrens (b) können alle üblicherweise für derartige elektrophile Substitutionsreaktionen verwendbaren Lösungsmittel eingesetzt werden. Vorzugsweise verwendet man die als Reagenzien infrage kommenden Säuren oder Gemische, wie beispielsweise Salpetersäure, Nitriersäure oder Sulfurylchlorid, gleichzeitig als Verdünnungsmittel. Es können gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel verwendet werden.

Als Katalysatoren oder Reaktionshilfsmittel zur Durchführung des erfindungsgemäßen Verfahrens (b) können ebenfalls die für derartige elektrophile Substitutionsreaktionen üblichen Reaktionshilfsmittel eingesetzt werden. Vorzugsweise verwendet man saure Katalysatoren, wie beispielsweise Schwefelsäure, Eisen-(III)-chlorid oder andere Lewis-Säuren oder auch Acetanhydrid.

Die Reaktionstemperaturen können bei der Durchführung des erfindungsgemäßen Verfahrens (b) in einem größeren Bereich variiert werden. Im allgemeinen arbeitet man bei Temperaturen zwischen -50 °C und +200 °C, vorzugsweise bei Temperaturen zwischen -20 °C und +150 °C.

Das erfindungsgemäße Verfahren (b) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (b) setzt man pro Mol substituierten Heterocyclyltriazindions der Formel (I) im allgemeinen 1,0 bis 10,0 Mol, vorzugsweise 1,0 bis 5,0 Mol elektrophiles Reagenz und gegebenenfalls 0,1 bis 10,0 Mol Katalysator oder Reaktionshilfsmittel ein.
Die Reaktionsdurchführung, Aufarbeitung und Isolierung der Reaktionsprodukte erfolgt nach allgemein bekannten Verfahren.

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes, oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen, Mais oder Soja einsetzen.

Darüberhinaus eignen sich die Wirkstoffe zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen.
Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiells, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus haloleucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio

hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Die erfindungsgemäßen Wirkstoffe zeichnen sich durch eine hohe insektizide Wirksamkeit aus. Sie lassen sich mit besonders gutem Erfolg zur Bekämpfung von pflanzenschädigenden Insekten, wie beispielsweise gegen die schwarze Bohnenblattlaus (Aphis fabae) einsetzen. Dabei zeigen die erfindungsgemäßen Wirkstoffe neben protektiven auch wurzelsystemische Eigenschaften.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, die bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil;

Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können bei der Verwendung als Herbizide als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei der Verwendung als Herbizide sowohl vor, als auch nach dem Auflaufen der Pflanzen appliziert werden.

Sie können auch vor der Saat in den Boden eingearbeitet werden.

Die angewandte Wirkstoffmenge kann bei der Verwendung als Herbizide in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die erfindungsgemäßen Wirkstoffe können bei der Anwendung als Insektizide ebenfalls in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können bei der Anwendung als Insektizide ferner in ihren handelsüblichen Formulierungen sowie in den aus diesen Formulierungen bereiteten Anwendungsformen in Mischung mit Synergisten vorliegen. Synergisten sind Verbindungen, durch die eine Wirkungssteigerung der Wirkstoffe erreicht wird, ohne daß der zugesetzte Synergist selbst aktiv wirksam sein muß.

Der Wirkstoffgehalt der aus den handelsüblichen Formulierungen bereiteten Anwendungsformen kann bei der Anwendung als Insektizide in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Herstellung und die Verwendung der erfindungsgemäßen Wirkstoffe geht aus den nachfolgenden Beispielen hervor.

Herstellungsbeispiele:

Beispiel 1:

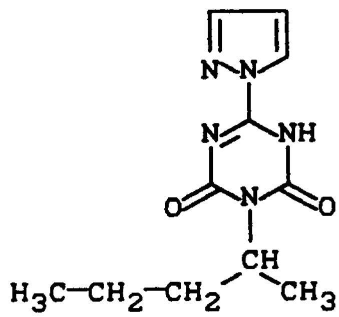

(Verfahren a)

Zu 4 g (0,0175 Mol) 1-(1-Methyl-1-butyl)-4-methylthio-1,3,5-(3H)-triazin-2,6-dion (vergleiche z. B. US 3,973,947) in 100 ml o-Dichlorbenzol gibt man bei Raumtemperatur 4,8 g (0,07 Mol) 1H-Pyrazol und erhitzt anschließend für eine Stunde auf Rückflußtemperatur. Zur Aufarbeitung wird die abgekühlte Reaktionsmischung mit Dichlormethan verdünnt, mit verdünnter Salzsäure pyrazolfrei gewaschen, über Magnesiumsulfat getrocknet und im Vakuum eingeengt. Der Rückstand wird aus Cyclohexan umkristallisiert.

Man erhält 1,8 g (41 % der Theroie) 1-(1-Methyl-1-butyl)-4-(1-pyrazolyl)-1,3,5-(3H)-triazin-2,6-dion vom Schmelzpunkt 99 °C bis 100 °C.

In entsprechender Weise und gemäß den allgemeinen Angaben zur Herstellung erhält man die folgenden substituierten Heterocyclyltriazindione der allgemeinen Formel (I):

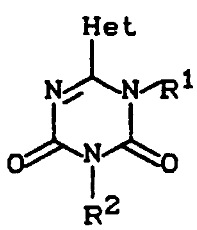

(I)

| Bsp.-Nr. | $R^1$ | $R^2$ | Het | physikalische Eigenschaften |
|---|---|---|---|---|
| 2 | H | $i\text{-}C_3H_7$ | 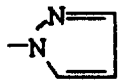 | Fp. 142° C |
| 3 | H | 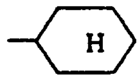 | 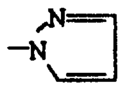 | Fp. 236° C |
| 4 | H | $i\text{-}C_3H_7$ | 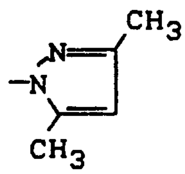 | Fp. 186° C |
| 5 | H | $i\text{-}C_3H_7$ | 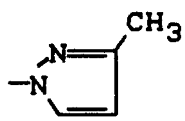 | Fp. 205° C |
| 6 | H | $n\text{-}C_3H_7$ | 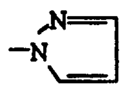 | Fp. 184° C |
| 7 | H | $CH_3$ | 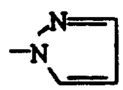 | Fp. >240° C |

| Bsp.-Nr. | $R^1$ | $R^2$ | Het | physikalische Eigenschaften |
|---|---|---|---|---|
| 8 | H | $n-C_4H_9$ | 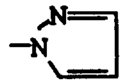 | Fp. 199 °C |
| 9 | H | $i-C_3H_7$ | 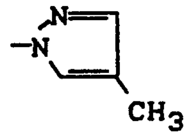 | Fp. 171 °C |
| 10 | H | $s-C_4H_9$ | 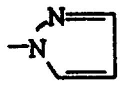 | Fp.>230 °C |
| 11 | H | 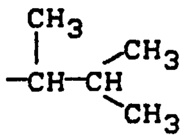 | 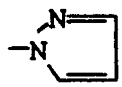 | Fp. 128-129° C |
| 12 | H | $-CH_2-CH=CH_2$ | 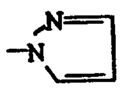 | Fp. > 230°C |
| 13 | H | 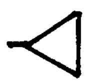 | 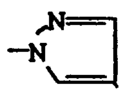 | Fp. 240-241°C |
| 14 | H | $C_2H_5$ | 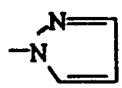 | Fp. 194-195°C |
| 15 | H | $-CH_2-C(CH_3)_3$ | 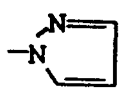 | Fp. 197-199°C |

| Bsp.-Nr. | $R^1$ | $R^2$ | Het | physikalische Eigenschaften |
|---|---|---|---|---|
| 16 | H | $i-C_3H_7$ | 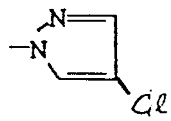 | Fp. 137°C |
| 17 | H | $i-C_3H_7$ | 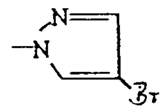 | Fp. 190°C |
| 18 | H | $i-C_3H_7$ | 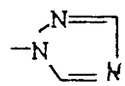 | Fp. 184°C |

Anwendungsbeispiele:

In den folgenden Anwendungsbeispielen wurde die nachstehend aufgeführte Verbindung als Vergleichssubstanz eingesetzt:

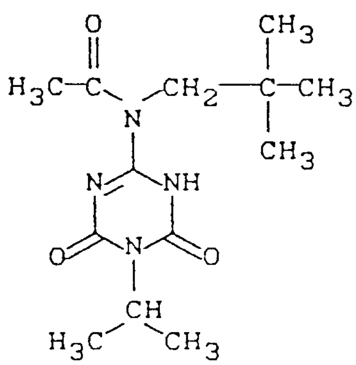

(A)

1-Isopropyl-4-[N-(2,2-dimethylpropyl)-acetamido]-1,3,5-(3H)-triazin-2,6-dion (vergleiche z.B. DE 26 03 180)

Beispiel A:

Post-emergence-Test

Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im

Vergleich zur Entwicklung der unbehandelten Kontrolle.
Es bedeuten:
O % = keine Wirkung (wie unbehandelte Kontrolle)
100 % = totale Vernichtung
Eine deutliche Überlegenheit in der Wirksamkeit ebenso wie in der Nutzpflanzenselektivität gegenüber dem Stand der Technik zeigt in diesem Test z.B. die Verbindung gemäß Herstellungsbeispiel 2.

Beispiel B:

Aphis-Test (systemische Wirkung)

Lösungsmittel: 7 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenblattlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattlaus abgetötet wurde.

Bei diesem Test zeigt z.B. die Verbindung 2 der Herstellungsbeispiele überlegene Wirksamkeit gegenüber dem Stand der Technik.

**Patentansprüche**

**1.** Substituierte Heterocyclyltriazindione der allgemeinen Formel (I)

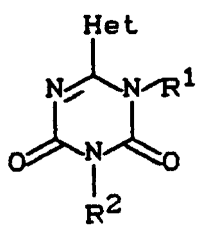

(I)

in welcher

$R^1$ für Wasserstoff oder für einen jeweils gegebenenfalls substituierten Rest der Reihe Alkyl, Alkenyl, Alkinyl oder Cycloalkyl steht,

$R^2$ für einen jeweils gegebenenfalls substituierten Rest der Reihe Alkyl, Alkenyl, Alkinyl oder Cycloalkyl steht und

Het für einen N-verknüpften, gegebenenfalls substituierten, ungesättigten, stickstoffhaltigen Heterocyclus steht, der gegebenenfalls weitere Heteroatome enthalten kann.

**2.** Substituierte Heterocyclyltriazindione der allgemeinen Formel (I) gemäß Anspruch 1, dadurch gekennzeichnet, daß

$R^1$ für Wasserstoff, für einen jeweils geradkettigen oder verzweigten Rest der Reihe Alkyl mit 1 bis 12 Kohlenstoffatomen, Alkenyl mit 2 bis 6 Kohlenstoffatomen, Alkinyl mit 2 bis 6 Kohlenstoffatomen, Alkoxyalkyl oder Alkylthioalkyl mit jeweils 1 bis 6 Kohlenstoffatomen in den einzelnen Alkylteilen, Halogenalkyl mit 1 bis 6 Kohlenstoffatomen und 1 bis 13 gleichen oder verschiedenen Halogenatomen, Halogenalkenyl mit 2 bis 6 Kohlenstoffatomen und 1 bis 11 gleichen oder verschiedenen Halogenatomen, Halogenalkinyl mit 2 bis 6 Kohlenstoffatomen und 1 bis 9 gleichen oder verschiedenen Halogenatomen
oder für gegebenenfalls einfach oder mehrfach, gleich oder verschieden substituiertes Cycloalkyl mit 3 bis 8 Kohlenstoffatomen steht, wobei als Cycloalkylsubstituenten infrage kommen: Halogen, geradkettiges oder verzweigtes Alkyl mit 1 bis 4 Kohlenstoffatomen oder

geradkettiges oder verzweigtes Halogenalkyl mit 1 bis 4 Kohlenstoffatomen und 1 bis 9 gleichen oder verschiedenen Halogenatomen,

$R^1$ außerdem für jeweils gegebenenfalls im Aryl- bzw. Heteroarylteil gegebenenfalls einfach oder mehrfach, gleich oder verschieden substituiertes Arylalkyl, Aryloxyalkyl, Arylthioalkyl oder Heteroarylalkyl mit jeweils 1 bis 6 Kohlenstoffatomen im geradkettigen oder verzweigten Alkylteil und jeweils 6 bis 10 Kohlenstoffatomen im Arylteil bzw. mit 2 bis 9 Kohlenstoffatomen und 1 bis 4 Heteroatomen - insbesondere Stickstoff, Sauerstoff und/oder Schwefel - im Heteroarylteil steht, wobei als Aryl bzw. Heteroarylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^2$ für einen jeweils geradkettigen oder verzweigten Rest der Reihe Alkyl, Alkoxy oder Alkylthio mit jeweils 1 bis 8 Kohlenstoffatomen, Alkenyl mit 2 bis 6 Kohlenstoffatomen, Alkinyl mit 2 bis 6 Kohlenstoffatomen, Halogenalkyl mit 1 bis 6 Kohlenstoffatomen und 1 bis 13 gleich oder verschiedenen Halogenatomen, Halogenalkenyl mit 2 bis 6 Kohlenstoffatomen und 1 bis 9 gleichen oder verschiedenen Halogenatomen, Halogenalkinyl mit 2 bis 6 Kohlenstoffatomen und 1 bis 9 gleichen oder verschiedenen Halogenatomen oder für gegebenenfalls einfach oder mehrfach, gleich oder verschieden substituiertes Cycloalkyl mit 3 bis 8 Kohlenstoffatomen steht, wobei als Cycloalkylsubstituenten infrage kommen:
Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen,

$R^2$ außerdem für jeweils gegebenenfalls im Aryl- bzw. Heteroarylteil gegebenenfalls einfach oder mehrfach, gleich oder verschieden substituiertes Arylalkyl, Aryloxyalkyl, Arylthioalkyl oder Heteroarylalkyl mit jeweils 1 bis 6 Kohlenstoffatomen im geradkettigen oder verzweigten Alkylteil und jeweils 6 bis 10 Kohlenstoffatomen im Arylteil bzw. mit 2 bis 9 Kohlenstoffatomen und 1 bis 4 Heteroatomen - insbesondere Stickstoff, Sauerstoff und/oder Schwefel - im Heteroarylteil steht, wobei als Aryl- bzw. Heteroarylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl und

Het für einen N-verknüpften, gegebenenfalls einfach oder mehrfach, gleich oder verschieden substituierten, einfach oder mehrfach ungesättigten, stickstoffhaltigen Heterocyclus mit 2 bis 9 Kohlenstoffatomen und gegebenenfalls 1 bis 4 weiteren Heteroatomen - insbesondere Stickstoff, Sauerstoff und/oder Schwefel - steht, wobei als Heterocyclylsubstituenten die bei $R^2$ genannten infrage kommen.

**3.** Substituierte Heterocyclyltriazindiole der allgemeinen Formel (I) gemäß Anspruch 1, dadurch gekennzeichnet, daß

$R^1$ für Wasserstoff, für einen jeweils geradkettigen oder verzweigten Rest der Reihe Alkyl mit 1 bis 10 Kohlenstoffatomen, Alkenyl mit 2 bis 5 Kohlenstoffatomen, Alkinyl mit 2 bis 5 Kohlenstoffatomen, Alkoxyalkyl oder Alkylthioalkyl mit jeweils 1 bis 4 Kohlenstoffatomen in den einzelnen Alkylteilen, Halogenalkyl mit 1 bis 4 Kohlenstoffatomen und 1 bis 9 gleichen oder verschiedenen Halogenatomen, Halogenalkenyl mit 2 bis 5 Kohlenstoffatomen und 1 bis 9 gleichen oder verschiedenen Halogenatomen, Halogenalkinyl mit 2 bis 5 Kohlenstoffatomen und 1 bis 7 gleichen oder verschiedenen Halogenatomen
oder für gegebenenfalls einfach bis fünffach, gleich oder verschieden substituiertes Cycloalkyl mit 3 bis 7 Kohlenstoffatomen steht, wobei als Cycloalkylsubstituenten infrage kommen:
Halogen, geradkettiges oder verzweigtes Alkyl mit 1 bis 3 Kohlenstoffatomen oder geradkettiges oder verzweigtes Halogenalkyl mit 1 bis 3 Kohlenstoffatomen und 1 bis 7 gleichen oder verschiedenen Halogenatomen,

$R^1$ außerdem für jeweils gegebenenfalls im Aryl- bzw. Heteroarylteil gegebenenfalls einfach bis fünffach, gleich oder verschieden substituiertes Arylalkyl, Aryloxyalkyl, Arylthioalkyl oder Heteroarylalkyl mit jeweils 1 bis 4 Kohlenstoffatomen im geradkettigen oder verzweigten Alkylteil und jeweils 6 oder 10 Kohlenstoffatomen im Arylteil bzw. mit 2 bis 9 Kohlenstoffatomen und 1 bis 3 Heteroatomen - insbesondere Stickstoff, Sauerstoff und/oder Schwefel - im Heteroarylteil steht, wobei als Aryl- bzw. Heteroarylsubstituenten jeweils infrage kommen:
Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 2 Kohlenstoffatomen und 1 bis 5 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis fünffach, gleich oder verschieden durch Halogen und/oder geradkettiges oder verzweigtes Alkyl mit 1 bis 3 Kohlenstoffatomen substituiertes Phenyl,

$R^2$ für einen jeweils geradkettigen oder verzweigten Rest der Reihe Alkyl, Alkoxy oder Alkylthio mit jeweils 1 bis 6 Kohlenstoffatomen, Alkenyl mit 2 bis 5 Kohlenstoffatomen, Alkinyl mit 2 bis 5 Kohlenstoffatomen, Halogenalkyl mit 1 bis 4 Kohlenstoffatomen und 1 bis 9 gleichen oder verschiedenen Halogenatomen, Halogenalkenyl mit 2 bis 5 Kohlenstoffatomen und 1 bis 9 gleichen oder verschiedenen Halogenatomen, Halogenalkinyl mit 2 bis 5 Kohlenstoffatomen und 1 bis 7 gleichen oder verschiedenen Halogenatomen oder für gegebenenfalls einfach bis fünffach, gleich oder verschieden substituiertes Cycloalkyl mit 3 bis 7 Kohlenstoffatomen steht, wobei als Cycloalkylsubstituenten infrage kommen:
Halogen, geradkettiges oder verzweigtes Alkyl mit 1 bis 3 Kohlenstoffatomen oder geradkettiges oder verzweigtes Halogenalkyl mit 1 bis 3 Kohlenstoffatomen und 1 bis 7 gleichen oder verschiedenen Halogenatomen,

$R^2$ außerdem für jeweils gegebenenfalls im Aryl- bzw. Heteroarylteil gegebenenfalls einfach bis fünffach, gleich oder verschieden substituiertes Arylalkyl, Aryloxyalkyl, Arylthioalkyl oder Heteroarylalkyl mit jeweils 1 bis 4 Kohlenstoffatomen im geradkettigen oder verzweigten Alkylteil und jeweils 6 oder 10 Kohlenstoffatomen im Arylteil bzw. mit 2 bis 9 Kohlenstoffatomen und 1 bis 3 Heteroatomen - insbesondere Stickstoff, Sauerstoff und/oder Schwefel - im Heteroarylteil steht, wobei als Aryl- bzw. Heteroarylsubstituenten jeweils infrage kommen:
Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 2 Kohlenstoffatomen und 1 bis 5 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis fünffach, gleich oder verschieden durch Halogen und/oder geradkettiges oder verzweigtes Alkyl mit 1 bis 3 Kohlenstoffatomen substituiertes Phenyl und

Het für einen N-verknüpften, gegebenenfalls einfach bis fünffach, gleich oder verschieden substituierten, einfach oder mehrfach ungesättigten, stickstoffhaltigen Heterocyclus mit 2 bis 9 Kohlenstoffatomen und gegebenenfalls 1 bis 3 weiteren Heteroatomen - insbesondere Stickstoff, Sauerstoff und/oder Schwefel - steht, wobei als Heterocyclylsubstituenten die bei $R^2$ genannten infrage kommen.

**4.** Substituierte Heterocyclyltriazindione der allgemeinen Formel (I) gemäß Anspruch 1, dadurch gekennzeichnet, daß

$R^1$ für Wasserstoff, für einen jeweils geradkettigen oder verzweigten Rest der Reihe Alkyl mit 1 bis 8 Kohlenstoffatomen, Alkenyl mit 2 bis 3 Kohlenstoffatomen, Alkinyl mit 2 bis 3 Kohlenstoffatomen, Alkoxyalkyl oder Alkylthioalkyl mit jeweils 1 bis 3 Kohlenstoffatomen in den einzelnen Alkylteilen, Halogenalkyl mit 1 oder 2 Kohlenstoffatomen und 1 bis 5 gleichen oder verschiedenen Halogenatomen, Halogenalkenyl mit 2 bis 3 Kohlenstoffatomen und 1 bis 5 gleichen oder verschiedenen Halogenatomen, Halogenalkinyl mit 2 bis 3 Kohlenstoffatomen und 1 bis 3 gleichen oder verschiedenen Halogenatomen
oder für jeweils gegebenenfalls einfach bis dreifach, gleich oder verschieden substituiertes Cyclopropyl, Cyclopentyl oder Cyclohexyl steht, wobei als Substituenten jeweils infrage kommen;

Fluor, Chlor, Brom, Methyl, Ethyl oder Trifluormethyl,

$R^1$ außerdem für jeweils gegebenenfalls im Phenyl- bzw. Heteroarylteil gegebenenfalls einfach bis dreifach, gleich oder verschieden substituiertes Phenylalkyl, Phenyloxyalkyl, Phenylthioalkyl oder Heteroarylalkyl mit jeweils 1 bis 2 Kohlenstoffatomen im Alkylteil und gegebenenfalls 2 bis 9 Kohlenstoffatomen und 1 bis 3 Heteroatomen - insbesondere Stickstoff, Sauerstoff und/oder Schwefel - im Heteroarylteil steht, wobei als Phenyl- bzw. Heteroarylsubstituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n-oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl,

$R^2$ für einen jeweils geradkettigen oder verzweigten Rest der Reihe Alkyl, Alkoxy oder Alkylthio mit jeweils 1 bis 4 Kohlenstoffatomen, Alkenyl mit 2 bis 3 Kohlenstoffatomen, Alkinyl mit 2 bis 3 Kohlenstoffatomen, Halogenalkyl mit 1 bis 2 Kohlenstoffatomen und 1 bis 5 gleichen oder verschiedenen Halogenatomen, Halogenalkenyl mit 2 bis 3 Kohlenstoffatomen und 1 bis 5 gleichen oder verschieden Halogenatomen, Halogenalkinyl mit 2 bis 3 Kohlenstoffatomen und 1 bis 3 gleichen oder verschiedenen Halogenatomen oder für jeweils gegebenenfalls einfach bis dreifach, gleich oder verschieden substituiertes Cyclopropyl, Cyclopentyl oder Cyclohexyl steht, wobei als Substituenten jeweils infrage kommen:

Fluor, Chlor, Brom, Methyl, Ethyl oder Trifluormethyl,

$R^2$ außerdem für jeweils gegebenenfalls im Phenyl- bzw. Heteroarylteil gegebenenfalls einfach bis dreifach, gleich oder verschieden substituiertes Phenylalkyl, Phenyloxyalkyl, Phenylthioalkyl oder Heteroarylalkyl mit jeweils 1 bis 2 Kohlenstoffatomen im geradkettigen oder verzweigten Alkylteil und gegebenenfalls 2 bis 9 Kohlenstoffatomen und 1 bis 3 Heteroatomen - insbesondere Stickstoff, Sauerstoff und/oder Schwefel - im Heteroarylteil steht, wobei als Phenyl- bzw. Heteroarylsubstituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl und

Het für jeweils gegebenenfalls einfach bis dreifach, gleich oder verschieden substituiertes, jeweils N-verknüpftes Pyrazolyl, Pyrrolyl, Imidazolyl, Triazolyl, Tetrazolyl, Indolyl, Indazolyl oder Benzimidazolyl steht, wobei als Heterocyclylsubstituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

**5.** Verfahren zur Herstellung von substituierten Heterocyclyltriazindionen der allgemeinen Formel (I)

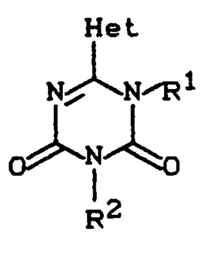

(I)

in welcher

$R^1$ für Wasserstoff oder für einen jeweils gegebenenfalls substituierten Rest der Reihe Alkyl,

Alkenyl, Alkinyl oder Cycloalkyl steht,

$R^2$ für einen jeweils gegebenenfalls substituierten Rest der Reihe Alkyl, Alkenyl, Alkinyl oder Cycloalkyl steht und

Het für einen N-verknüpften, gegebenenfalls substituierten, ungesättigten, stickstoffhaltigen Heterocyclus steht, der gegebenenfalls weitere Heteroatome enthalten kann,

dadurch gekennzeichnet, daß man

a) 4-Alkylthio-triazindione der Formel (II),

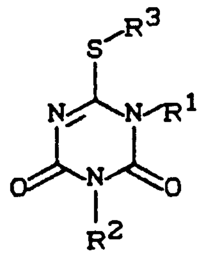

(II)

in welcher

$R^1$ und $R^2$ die oben angegebene Bedeutung haben

und

$R^3$ für Alkyl steht,

mit Heterocyclen der Formel (III),

Het-H(III)

in welcher

Het die oben angegebene Bedeutung hat,

gegebenenfalls in Gegenwart eines Verdünnungsmittels umsetzt und gegebenenfalls anschließend

b) die so erhältlichen substituierten Heterocyclyltriazindione der Formel (I),

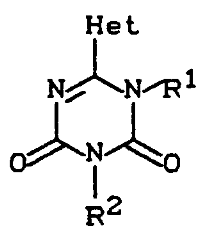

(I)

in welcher

$R^1$, $R^2$ und Het die oben angegebene Bedeutung haben,

mit elektrophilen Reagenzien im Heterocyclylteil des Substituenten Het gegebenenfalls in Gegenwart eines Verdünnungsmittels und gegebenenfalls in Gegenwart eines Reaktionshilfsmittels substituiert.

**6.** Herbizide und insektizide Mittel, gekennzeichnet durch einen Gehalt an mindestens einem substituierten Heterocyclyltriazindion der Formel (I) gemäß dem Anspruch 1.

**7.** Verfahren zur Bekämpfung von unerwünschten Pflanzen, dadurch gekennzeichnet, daß man substituierte Heterocyclyltriazindione der Formel (I) gemäß dem Anspruch 1 auf unerwünschte Pflanzen und/oder ihren Lebensraum einwirken läßt.

**8.** Verwendung von substituierten Heterocyclyltriazindionen der Formel (I) gemäß dem Anspruch 1 zur Bekämpfung von unerwünschten Pflanzen.

**9.** Verfahren zur Herstellung von herbiziden und insektiziden Mitteln, dadurch gekennzeichnet, daß man substituierte Heterocyclyltriazindione der Formel (I) gemäß dem Anspruch 1 mit Streckmitteln und/oder

oberflächenaktiven Substanzen vermischt.

**10.** Verfahren zur Bekämpfung von unerwünschten Insekten, dadurch gekennzeichnet, daß man substituierte Heterocyclyltriazindione der Formel (I) gemäß dem Anspruch 1 auf unerwünschte Insekten und/oder ihren Lebensraum einwirken läßt.

**11.** Verwendung von substituierten Heterocyclyltriazindionen der Formel (I) gemäß dem Anspruch 1 zur Bekämpfung von unerwünschten Insekten.

Europäisches Patentamt

# EUROPÄISCHER RECHERCHENBERICHT

Nummer der Anmeldung

EP 92 12 0820

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | DE-A-2 603 180 (IMPERIAL CHEMICAL INDUSTRIES LTD)<br>* Ansprüche *<br>--- | 1,6 | C07D403/04<br>A01N43/64 |
| A | CHEMICAL ABSTRACTS, vol. 95, 1981, Columbus, Ohio, US;<br>abstract no. 150711h, 'Triazine-2,6,-dione derivatives'<br>Seite 677 ;<br>* Zusammenfassung *<br>& JP 81 51,462 (Nissan Chemical Industires, Ltd.)<br>----- | 1,6 | |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 21 JANUAR 1993 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)